# EUROPEAN PATENT APPLICATION

(11) **EP 3 255 961 A1**
(43) Date of publication of application: **13.12.2017**
(21) Application number: 17174561.5
(22) Date of filing: 06.06.2017
(51) Int. Cl.: H05H 1/24, C02F 1/78

(54) **PLASMA LIQUID GENERATING DEVICE**

(30) Priority: 06.06.2016 TW 105117873
(71) Applicant: Amsalp Biomedical Co., Ltd., Taipei 105 (TW)
(72) Inventor: LU, Jen-Chieh, 220 Taipei (TW); HSIAO, Yung-Leng, 621 Chiayi Country (TW); LU, Yin-Lin, 802 Kaohsiung City (TW)
(74) Representative: dompatent von Kreisler Selting Werner - Partnerschaft von Patent- und Rechtsanwälten mbB

(57) **Abstract**

A plasma liquid generating device includes a plasma generating module, a driving circuit, an adjust-controlling module and a mixing structure. The driving circuit is coupled with the plasma generating module and configured to drive the plasma generating module to generate first type plasma particles and second type plasma particles. The adjust-controlling module is coupled with the driving circuit and configured to control the driving circuit to adjust a proportion of the first type plasma particles and the second type plasma particles generated by the plasma generating module. The mixing structure connects with the plasma generating module and configured to mix the first type plasma particles, the second type plasma particles and a liquid so as to produce a plasma liquid.

## Description

### BACKGROUND OF THE INVENTION

### Field of Invention

The present disclosure relates to a generating device and, in particular, to a plasma liquid generating device.

### Related Art

Plasma technologies have been widely applied to various industries. Most maturated plasma technologies must be carried out under the vacuum environment. However, the vacuum processes have some drawbacks such as time consuming for vacuuming, high costs for preparing and repairing the vacuum equipment, size limitation of the chamber, difficult to performing continuous processing, and etc. Recently, the atmospheric-pressure plasma technology has been introduced, and it doesn't have the above limitations. In more specific, the atmospheric-pressure plasma technology has lower costs for preparing and repairing the equipment, is capable of rapidly generating plasma, and is suitable for continuous processing. Thus, the atmospheric-pressure plasma technology has become one of the most popular topics. Besides, the atmospheric-pressure plasma technology is potential to the cosmetology and medical applications.

Taking cosmetology and medical applications for example, in order to restore the elasticity and glory of the skin, various kinds of cosmetology have been developed. For example, the first generation of laser technology, pulsed light, and radio waves are applied in the beauty care field. Nowadays, the plasma activation is not only for sterilization and improving skin collagens, but also re-regenerating elastic fibroblasts, thereby giving the experiencers an unprecedented new experience. However, the current plasma activation therapy can only be carried out in the medical institutions, and most people can't experience the plasma activation treatment in their daily lives. Most people can't produce plasma liquid themselves in their daily lives.

### SUMMARY OF THE INVENTION

An objective of the present disclosure is to provide a plasma liquid generating device that can generate a plasma liquid in daily lives.

In one embodiment, the present disclosure discloses a plasma liquid generating device, which includes a plasma generating module, a driving circuit, an adjust-controlling module, and a mixing structure. The driving circuit is coupled with the plasma generating module and configured to drive the plasma generating module to generate first type plasma particles and second type plasma particles. The adjust-controlling module is coupled with the driving circuit and configured to control the driving circuit to adjust a generation proportion of the first type plasma particles and the second type plasma particles generated by the plasma generating module. The mixing structure connects with the plasma generating module and is configured to mix the first type plasma particles, the second type plasma particles and a liquid so as to produce a plasma liquid.

In one embodiment, the first type plasma particles are ozone, and the second type plasma particles are nitrate ions.

In one embodiment, the driving circuit is configured to output a driving voltage to the plasma generating module. When the driving voltage has a high frequency, the generated nitrate ions are more than the generated ozone in the generation proportion. When the driving voltage has a low frequency, the generated ozone is more than the generated nitrate ions in the generation proportion.

In one embodiment, the plasma liquid generating device further includes a power module configured to output a supply voltage to the driving circuit.

In one embodiment, the driving circuit includes a voltage frequency adjustor, a voltage convertor and a booster. The voltage frequency adjustor is configured to generate a modulated frequency according to an adjusting signal outputted from the adjust-controlling module. The voltage convertor is coupled with the power module and the voltage frequency adjustor, and configured to output the driving voltage according to the supply voltage and the modulation frequency. The booster is configured to boost the driving voltage and output the driving voltage to the plasma generating module.

In one embodiment, the power module includes a hydroelectric generator, and the liquid flows through the hydroelectric generator so as to enable the hydroelectric generator to output the supply voltage.

In one embodiment, the adjust-controlling module includes a controller such as a touch screen or a knob.

In one embodiment, the plasma generating module includes at least an atmospheric-pressure plasma generator, which includes a first electrode and a second electrode disposed opposite to the first electrode.

In one embodiment, the first electrode has a plate shape, the second electrode has at least a hole, and the first type plasma particles and the second type plasma particles flow into the mixing structure through the hole.

In one embodiment, the mixing structure includes a Venturi tube and an inlet and an outlet connecting to the Venturi tube, and the second electrode is disposed on the Venturi tube close to the outlet.

In one embodiment, the plasma generating module includes a first atmospheric-pressure plasma generator and a second atmospheric-pressure plasma generator. The first atmospheric-pressure plasma generator is configured to generate a group of plasma particles mainly containing the first type plasma particles. The second atmospheric-pressure plasma generator is configured to generate a group of plasma particles mainly containing the second type plasma particles.

In one embodiment, the plasma liquid generating device further includes a waterproof housing for accommodating the plasma generating module, the driving circuit and the mixing structure, and the adjust-controlling module is disposed on the waterproof housing.

In one embodiment, the disclosure discloses a compact automatic plasma liquid generating device, which includes at least an atmospheric-pressure plasma generator, a hydroelectric generator, a driving circuit, an adjust-controlling module, a mixing structure and a waterproof housing. The hydroelectric generator is configured to output a supply voltage based on a liquid flowing therethrough. The driving circuit is coupled with the atmospheric-pressure plasma generator. The driving circuit is configured to transform the supply voltage into a high voltage, and configured to drive the atmospheric-pressure plasma generator to generate ozone plasma particles and nitrate ion plasma particles. The adjust-controlling module is coupled with the driving circuit and configured to control the driving circuit to adjust a generation proportion of the ozone plasma particles and the nitrate ion plasma particles generated by the atmospheric-pressure plasma generator. The mixing structure connects with the atmospheric-pressure plasma generator and is configured to mix the ozone plasma particles, the nitrate ion plasma particles, and the liquid so as to produce a plasma liquid. The waterproof housing is configured for accommodating the atmospheric-pressure plasma generator, the hydroelectric generator, the driving circuit and the mixing structure, and the adjust-controlling module is disposed on the waterproof housing.

In one embodiment, when the high voltage has a high frequency, the generated nitrate ion plasma particles are more than the generated ozone plasma particles in the generation proportion. When the high voltage has a low frequency, the generated ozone plasma particles are more than the generated nitrate ion plasma particles in the generation proportion.

In one embodiment, the driving circuit includes a voltage frequency adjustor, a voltage convertor and a booster. The voltage frequency adjustor generates a modulated frequency according to an adjusting signal outputted from the adjust-controlling module. The voltage convertor is coupled with the hydroelectric generator and the voltage frequency adjustor and outputs the high voltage according to the supply voltage and the modulation frequency. The booster boosts the high voltage and outputs the high voltage to the hydroelectric generator.

In one embodiment, the plasma generating module includes at least an atmospheric-pressure plasma generator. The atmospheric-pressure plasma generator includes a first electrode and a second electrode, which are disposed opposite to each other.

In one embodiment, the first electrode has a plate shape, and the second electrode has at least a hole. The ozone plasma particles and the nitrate ion plasma particles flow into the mixing structure through the hole.

In one embodiment, the mixing structure includes a Venturi tube and an inlet and an outlet connecting to the Venturi tube, and the second electrode is disposed on the Venturi tube close to the outlet.

In one embodiment, the atmospheric-pressure plasma generator includes a first atmospheric-pressure plasma generator and a second atmospheric-pressure plasma generator. The first atmospheric-pressure plasma generator is configured to generate a group of plasma particles mainly containing the ozone plasma particles. The second atmospheric-pressure plasma generator is configured to generate a group of plasma particles mainly containing the nitrate ion plasma particles.

In one embodiment, an atmospheric-pressure plasma liquid generating device comprises at least an atmospheric-pressure plasma generator, a driving circuit and a mixing structure. The atmospheric-pressure plasma generator comprises an air inlet, a chamber and at least a plasma outlet. The driving circuit is coupled with the atmospheric-pressure plasma generator to drive the atmospheric-pressure plasma generator to generate plasma particles in the chamber. The mixing structure comprises a flow channel and a plasma inlet. The plasma inlet communicates with the plasma outlet and the flow channel. When a liquid flows through the flow channel, the plasma particles are sucked from the chamber through the plasma outlet and the plasma inlet to the flow channel and mixed in the liquid so as to produce a plasma liquid, and atmospheric-pressure air is sucked through the air inlet into the chamber.

In one embodiment, the atmospheric-pressure plasma generator comprises a first electrode and a second electrode, the first electrode is disposed opposite to the second electrode, the first electrode has a plate shape, and the second electrode has at least a hole as the plasma outlet.

In one embodiment, the mixing structure comprises a Venturi tube and an inlet and an outlet connecting to the Venturi tube, and the second electrode is disposed on the Venturi tube close to the outlet.

In one embodiment, the driving circuit is configured to output a driving voltage to the atmospheric-pressure plasma generator, and the plasma particles comprises ozone plasma particles and nitrate ion plasma particles. When the driving voltage has a high frequency, the generated nitrate ions are more than the generated ozone. When the driving voltage has a low frequency, the generated ozone is more than the generated nitrate ions. The atmospheric-pressure plasma liquid generating device further comprises an adjust-controlling module coupled with the driving circuit and configured to control the driving circuit to adjust a generation proportion of the ozone plasma particles and the nitrate ion plasma particles generated in the chamber by the atmospheric-pressure plasma generator.

In one embodiment, the atmospheric-pressure plasma liquid generating device further comprises a hydroelectric generator configured to output a supply voltage based on the liquid flowing therethrough. The driving circuit is coupled with the atmospheric-pressure plasma generator and the hydroelectric generator, configured to transform the supply voltage into a high voltage, and configured to drive the atmospheric-pressure plasma generator to generate the plasma particles.

In one embodiment, the atmospheric-pressure plasma liquid generating device further comprises a power module configured to output a supply voltage to the driving circuit. The driving circuit comprises a voltage frequency adjustor, a voltage convertor and a booster. The voltage frequency adjustor is configured to generate a modulated frequency according to an adjusting signal outputted from the adjust-controlling module. The voltage convertor is coupled with the power module and the voltage frequency adjustor and configured to output the driving voltage according to the supply voltage and the modulation frequency. The booster is configured to boost the driving voltage and output the driving voltage to the plasma generating module.

In one embodiment, the power module comprises a hydroelectric generator, and the liquid flows through the hydroelectric generator so as to enable the hydroelectric generator to output the supply voltage.

In one embodiment, the atmospheric-pressure plasma liquid generating device comprises a plurality of the atmospheric-pressure plasma generators. One of the atmospheric-pressure plasma generators is configured to generate a group of plasma particles mainly containing a first type plasma particles. Another one of the atmospheric-pressure plasma generators is configured to generate a group of plasma particles mainly containing a second type plasma particles.

In one embodiment, the atmospheric-pressure plasma liquid generating device further comprises a waterproof housing for accommodating the plasma generating module, the driving circuit and the mixing structure.

As mentioned above, in the plasma liquid generating device, the driving circuit is coupled with the plasma generating module and drives the plasma generating module to generate first type plasma particles and second type plasma particles. Besides, the adjust-controlling module is coupled with the driving circuit and controls the driving circuit to adjust a generation proportion of the first type plasma particles and the second type plasma particles generated by the plasma generating module. The mixing structure mixes the first type plasma particles, the second type plasma particles and a liquid so as to produce a plasma liquid. Accordingly, the plasma liquid generating device can generate a plasma liquid, so that the users may produce plasma liquid in their daily lives.

### BRIEF DESCRIPTION OF THE DRAWINGS

The invention will become more fully understood from the detailed description and accompanying drawings, which are given for illustration only, and thus are not limitative of the present invention, and wherein:
FIG. 1 is a block diagram of a plasma liquid generating device according to an embodiment;
FIG. 2 is another block diagram of the plasma liquid generating device according to an embodiment;
FIGS. 3 and 4 are schematic diagrams showing a plasma generating module and a mixing structure;
FIGS. 5 and 6 are schematic diagrams showing applications of the plasma liquid generating device;
FIG. 7 is a block diagram of another plasma liquid generating device according to an embodiment; and
FIG. 8 is a schematic diagram showing a plasma generating module and a mixing structure.

### DETAILED DESCRIPTION OF THE INVENTION

The embodiments of the disclosure will be apparent from the following detailed description, which proceeds with reference to the accompanying drawings, wherein the same references relate to the same elements. Moreover, the drawings of all implementation are schematic, and they do not mean the actual size and proportion. The terms of direction recited in the disclosure, for example up, down, left, right, front, or rear, only define the directions according to the accompanying drawings for the convenience of explanation but not for limitation. The names of elements and the wording recited in the disclosure all have ordinary meanings in the art unless otherwise stated. Therefore, a person skilled in the art can unambiguously understand their meanings.

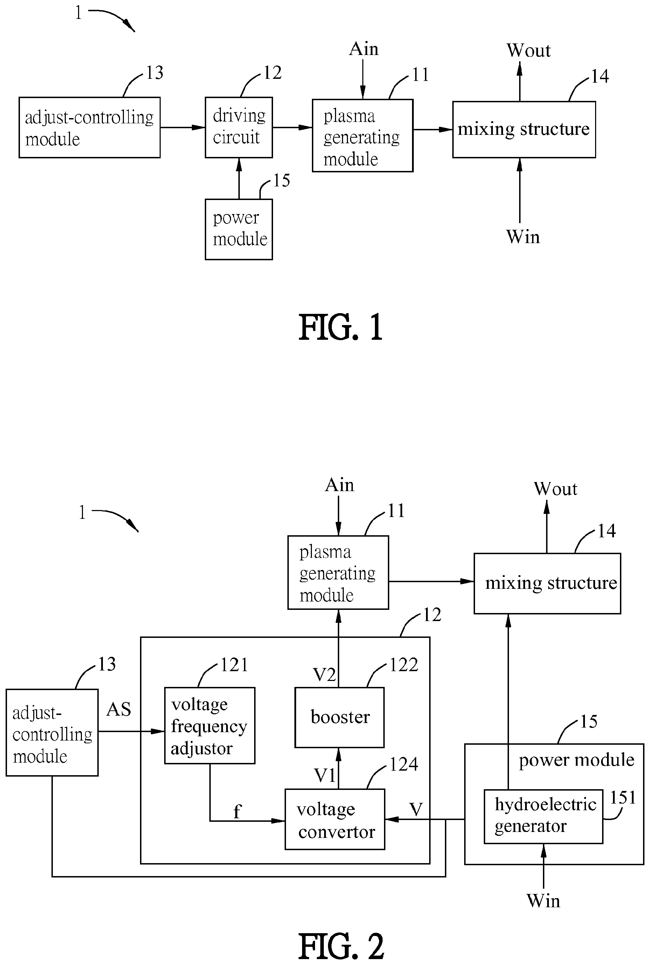

FIG. 1 is a block diagram of a plasma liquid generating device 1 according to an embodiment.

Referring to FIG. 1, the plasma liquid generating device 1 includes a plasma generating module 11, a driving circuit 12, an adjust-controlling module 13, and a mixing structure 14. In addition, the plasma liquid generating device 1 further includes a power module 15.

The plasma generating module 11 includes at least one plasma generator, which can ionize the entered air Ain so as to at least generate first type plasma particles and second type plasma particles. The entered air majorly contains oxygen and nitrogen. The plasma generator can ionize the oxygen and nitrogen so as to generate a plasma containing ozone (O₃), nitrate ions (NO₃⁻), and the likes. To be noted, the first type plasma particles is, for example but not limited to, ozone (O₃), and the second type plasma particles is, for example but not limited to, nitrate ions (NO₃⁻). Ozone (O₃) can be utilized for sterilization, and nitrate ions (NO₃⁻) have slightly acidic for removing the stratum corneum of skin. When the water containing these types of plasma particles (ozone and nitrate ions), it can provide cosmetic and sterilization functions and can be easily used.

The plasma generating module 11 is carried out based on the atmospheric-pressure plasma technology, which can increase the voltage to thousands volts or more. Accordingly, the electrons in the air can collide for multiple times within a limited space so as to accumulate sufficient electricity for ionizations, thereby generating the desired plasma. The atmospheric-pressure plasma technology can generate plasma under atmospheric pressure without the vacuum chamber and pump applied in the low-pressure plasma generation technology. Thus, the atmospheric-pressure plasma technology is more economic and efficiency, so that it can be applied in the general consumer products.

The plasma generator is, for example, an atmospheric-pressure plasma generator based on the technology of plasma jet, dielectric barrier discharge (DBD), corona discharge, plasma torch, or the likes. In this embodiment, the plasma generator generates the plasma based on, for example but not limited to, the DBD technology. In more details, the DBD technology utilizes high voltage and introduces an isolation plate between the electrodes for stabling the plasma.

The driving circuit 12 is coupled with the plasma generating module 11 and drives the plasma generating module 11 to generate first type plasma particles and second type plasma particles. The adjust-controlling module 13 is coupled with the driving circuit 12 and controls the driving circuit 12 to adjust a generation proportion of the first type plasma particles and the second type plasma particles generated by the plasma generating module 11. In other words, the user can optionally operate the adjust-controlling module 13 to adjust the settings of the driving signals (e.g. frequency, voltage, intensity or waveform) for adjusting a generation proportion of the first type plasma particles and the second type plasma particles generated by the plasma generating module 11. Accordingly, the proportions of the plasma particles in the plasma liquid can be controlled by the user.

In this embodiment, the first type plasma particles are ozone (O₃),and the second type plasma particles are nitrate ions (NO₃⁻). The plasma generating module 11 can generate the plasma particles of different proportions based on different driving signals. That is, the user can operate the adjust-controlling module 13 to adjust the settings or parameters of the plasma generating module 11, thereby adjusting the generation proportion of the plasma particles. For example, when the user wants to obtain a stronger sterilization function, he/she can operate the adjust-controlling module 13 to increase the proportion of ozone in the plasma particles, thereby mixing more ozone into the plasma liquid. Otherwise, when the user needs a function of removing stratum corneum, he/she can operate the adjust-controlling module 13 to increase the proportion of nitrate ions in the plasma particles, thereby mixing more nitrate ions into the plasma liquid.

In addition, the adjust-controlling module 13 may include a controller such as a touch screen or a knob, and the controller can individually carry out the function of the adjust-controlling module 13. When the controller is a touch screen, the user can directly operate the touch screen to control the adjust-controlling module 13 to output the adjust signal. When the controller is a knob, the user can tune the knob to control the adjust-controlling module 13 to output the adjust signal. Then, the adjust signal is inputted to the driving circuit 12 for adjusting the settings of the driving signal. In addition, the driving circuit 12 can control the plasma generating module 11 according to the adjust signal so as to adjust a generation proportion of the first type plasma particles and the second type plasma particles.

The mixing structure 14 is a liquid-gas mixing structure. The mixing structure 14 connects with the plasma generating module 11 and is configured to mix the first type plasma particles, the second type plasma particles and a liquid so as to produce a plasma liquid. In this embodiment, the liquid is, for example, water. As shown in figures, water Win enters the mixing structure 14 and is then mixed with the plasma particles to form a plasma liquid. Then, the plasma liquid Wout leaves the mixing structure 14, and the user can properly use the plasma liquid. The mixing structure 14 can introduce the gas containing the plasma particles, which are generated by the plasma generating module 11, into the liquid. When introducing the gas into the liquid, some bubbles will be generated in the liquid. Accordingly, the plasma liquid is water containing at least two types of plasma particles and bubbles, and the user can directly use the plasma liquid for experiencing the plasma activation treatment.

The power module 15 is a power supply source configured to output a supply voltage to the driving circuit 12. The power module 15 can output a DC voltage or an AC voltage. Besides, the power module 15 may further include a transformer, a frequency converter, a rectifier or an inverter depending on the required voltage. In addition, if the adjust-controlling module 13 includes an electronic component, the power module 15 can also output the supply voltage to the adjust-controlling module 13.

FIG. 2 is another block diagram of the plasma liquid generating device 1 according to the embodiment.

In this embodiment, the power module 15 includes a hydroelectric generator 151. Before being mixed with the plasma particles, the liquid flows through the hydroelectric generator 151 so as to enable the hydroelectric generator 151 to output the supply voltage V. In more detailed, the water Win enters the hydroelectric generator 151 and pushes the impeller of the hydroelectric generator 151 to generate electricity energy. In practice, the user can easily plug the water pipe to the inlet of the hydroelectric generator 151 and turn on the water valve, and then the water can flow into the hydroelectric generator 151 to enable the hydroelectric generator 151 to output voltage to other house equipment. For example, the hydroelectric generator 151 may output a supply voltage V (e.g. DC 12 Volts and 10 Watts) for driving the driving circuit 12 and the adjust-controlling module 13. In different embodiments, the power module 15 may not include the hydroelectric generator 151, but be activated by city power.

In addition, the plasma liquid generating device 1 further includes a housing for accommodating the plasma generating module 11, the driving circuit 12, the mixing structure 14 and the power module 15. In this embodiment, the hydroelectric generator 151 is a micro electricity generator, and the plasma generating module 11 is a small-sized dielectric barrier discharge (DBD) device. The housing is a waterproof housing. Accordingly, the plasma liquid generating device 1 has a compact size and can generate electricity by itself (the external power supply is unnecessary). Thus, the plasma liquid generating device 1 can be installed in the bathroom or applied in cleaning. In this case, the adjust-controlling module 13 is disposed on the housing, and the user can directly operate it.

In this embodiment, the driving circuit 12 includes a voltage frequency adjustor 121, a voltage convertor 124, and a booster 122. The voltage frequency adjustor 121 generates a modulated frequency f according to an adjusting signal AS outputted from the adjust-controlling module 13. The voltage convertor 124 is coupled with the power module 15 and the voltage frequency adjustor 121, and outputs the driving voltage (a first voltage V1) according to the supply voltage V and the modulation frequency f. The booster 122 is configured to boost the driving voltage (the first voltage V1) and outputting a second voltage V2 to the plasma generating module 11.

In this embodiment, the plasma generating module 11 can generate the plasma particles of different proportions according to different modulation frequencies f. The driving voltages of different frequencies can control the plasma generating module 11 to generate the first type plasma particles (O₃) and the second type plasma particles (NO₃⁻) of different proportions. For example, when the driving voltage (the second voltage V2) has a high frequency, the content of nitrate ions is more than the content of ozone in the generating proportion of the generated plasma particles. Alternatively, when the driving voltage (the second voltage V2) has a low frequency, the content of ozone is more than the content of nitrate ions in the generating proportion of the generated plasma particles. The invention is not limited thereto.

For example, the generated amount of ozone is not varied as the frequency of the driving voltage changes, but the amount of the ionized nitrogen increases as the frequency of the driving voltage increases. In this case, when the frequency of the driving voltage (the second voltage V2) is 0.5 kHz ∼ 5 kHz, the content of ozone is more than the content of nitrate ions in the generating proportion of the generated plasma particles. In practice, the voltage is less than 5kV. In addition, when the frequency of the driving voltage (the second voltage V2) is greater than 10 kHz, the content of nitrate ions is increased (compared with the lower frequency). When the frequency of the driving voltage is 15 kHz, the ionization rate of nitrogen is reduced. However, when the frequency of the driving voltage increases, the ionization rate of nitrogen is also increased. That is, in a higher frequency of the driving voltage (e.g. the frequency of the driving voltage is greater than 15 kHz), it is possible to generate more nitrate ions and the content of nitrate ions is more than the content of ozone.

In addition, the DBD technology needs high AC voltage configured to generate the desired plasma. In this embodiment, the DC-to-AC voltage convertor 124 can convert the DC supply voltage V outputted from the hydroelectric generator 151 to the AC first voltage C1. Then, the booster 122 boosts the first voltage V1 to output the second voltage V2 (high voltage) to the plasma generating module 11. The required high voltage value of the plasma generating module 11 depends on the types of the first type plasma particles and the second type plasma particles. The operation frequency of the plasma generating module 11 is 1∼40kHz, and the peak-to-peak voltage values can be thousands or more.

The adjust-controlling module 13 can output the adjusting signal AS for controlling the generating proportion of the first type plasma particles and the second type plasma particles generated by the plasma generating module 11. Accordingly, it is possible to adjust the proportion of the first type plasma particles and the second type plasma particles mixed in water.

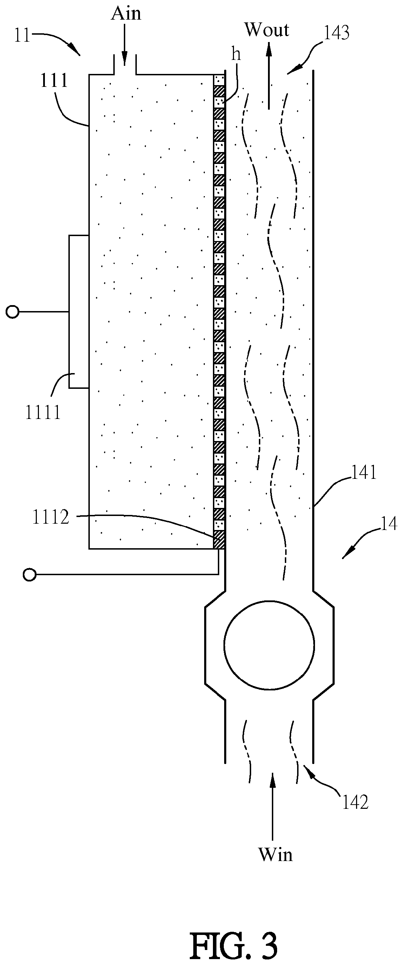

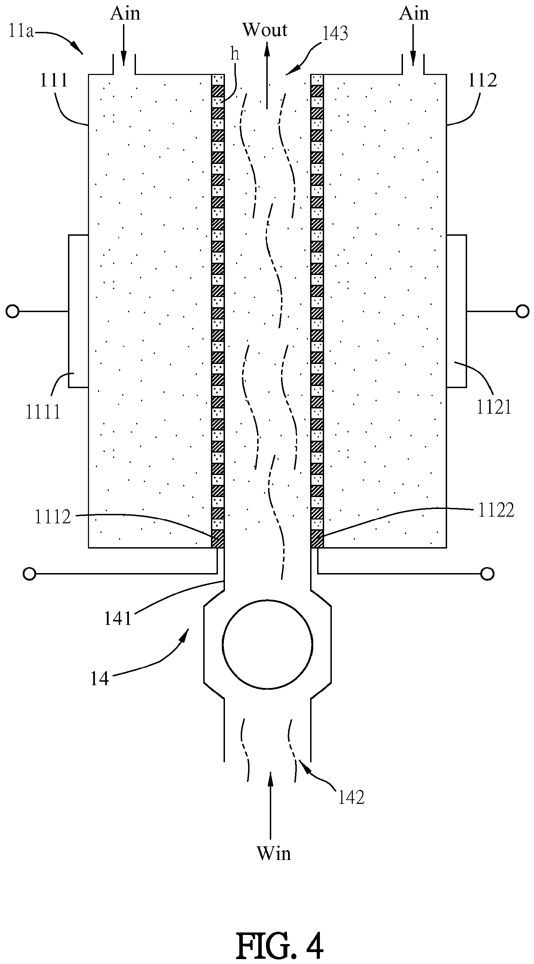

FIGS. 3 and 4 are schematic diagrams showing a plasma generating module 11 and a mixing structure 14.

As shown in FIG. 3, the plasma generating module 11 includes a plasma generator 111 (e.g. an atmospheric-pressure plasma generator). The plasma generator 111 includes a first electrode 1111 and a second electrode 1112 disposed opposite to the first electrode 1111. The AC voltage (driving voltage) can be inputted to the first electrode 1111 and the second electrode 1112 so as to generate a high electric field between the first electrode 1111 and the second electrode 1112. Air can be introduced into the chamber between the first electrode 1111 and the second electrode 1112, so that the air can be ionized in the high electric field to generate the first type plasma particles (O₃) and the second type plasma particles (NO₃⁻). The first electrode 1111 has a plate shape, and the second electrode 1112 has at least one hole h. In this embodiment, the second electrode 1112 is a mesh electrode having a plurality of holes h. In addition, the second electrode 1112 can fit to the shape of the mixing structure 14, and the second electrode 1112 and the mixing structure 14 are connected to each other. The first type plasma particles and the second type plasma particles, which are generated by the plasma generator 111, flow into the pipes of the mixing structure 14 through the holes h. Accordingly, the air inlet amount and the amount of the generated plasma can be controlled by the voltage difference between the first electrode 1111 and the second electrode 1112, the size of the holes h, the water flow speed, and the amount of the water.

In this embodiment, the mixing structure 14 includes a Venturi tube 141, an inlet 142, and an outlet 143. The inlet 142 and the outlet 143 are connected to the Venturi tube 141, and the second electrode 1112 is disposed on the Venturi tube 141 close to the outlet 143. The Venturi tube 141 also has a hole disposed close to the outlet 143 and corresponding to the holes h. Accordingly, the gas containing the first type plasma particles (O₃) and the second type plasma particles (NO₃⁻) can enter the Venturi tube 141 through the hole. The Venturi tube 141 can be operated based on Bernoulli's principle. When water flows into the inlet 142 and the outlet 143, the sectional area of the pipe are changed (from large to small), so that the flow speed is increased and the pressure is decreased. This configuration can form a negative pressure around the outlet 143 for sucking the gas containing the first type plasma particles (O₃) and the second type plasma particles (NO₃⁻) into the Venturi tube 141 through the hole. Then, these plasma particles can be resolved in water. The amount of air entering the plasma generator 111 depends on the amount of air containing the plasma particles being sucked into the Venturi tube 141. When the flow speed and amount of water increase, the amount of air entering into the plasma generator 111, the amount of plasma generated by the plasma generator 111, and the amount of air containing the plasma particles being sucked into the Venturi tube 141 are also increased. In other words, when the amount of water increases, the amounts of plasma particles and plasma liquid are also increased.

In addition, the plasma generating module may include a plurality of plasma generators. As shown in FIG. 4, the plasma generating module 11 a includes two plasma generators 111 and 112. The second electrodes 1112 and 1122 of the plasma generators 111 and 112 are disposed at two sides of the Venturi tube 141 close to the outlet 143. The configuration of two plasma generators 111 and 112 can generate more plasma particles, so that the water Wout (plasma liquid) exits from the outlet 143 contains more plasma particles.

To be noted, an energy stone can be installed at the outlet 143 of the mixing structure 14, so that the plasma liquid can flow through the energy stone before applying to the user. Herein, the energy stone can vibrate the water so as to turn the normal water into an active water, which has higher oxygen content. In one embodiment, the energy stone is, for example, a maifan stone, which can release minerals and absorb toxic and harmful substances, such as heavy metal ions (e.g. Pb, Hg, Cr, Cd, As), organic substances, bacteria, for purifying water.

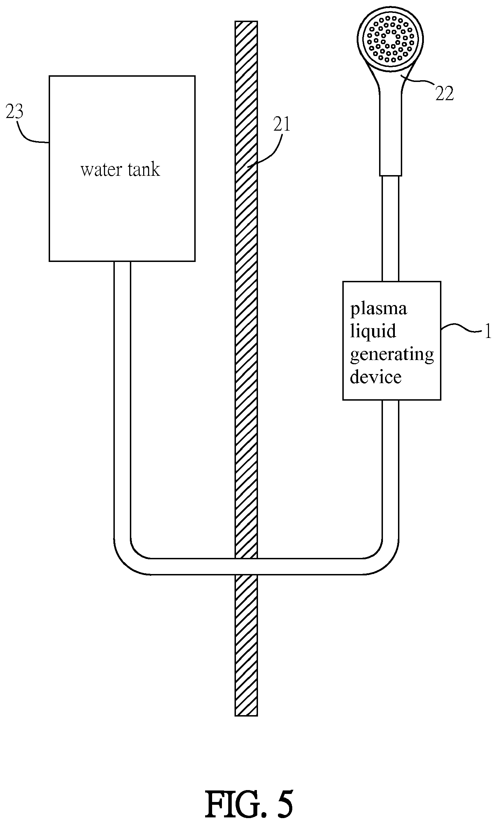

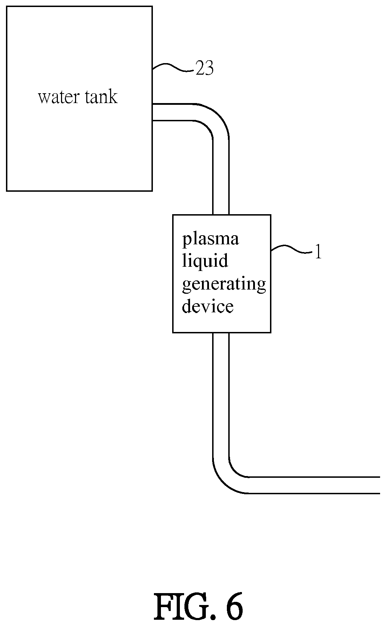

FIGS. 5 and 6 are schematic diagrams showing applications of the plasma liquid generating device.

As shown in FIG. 5, in one embodiment, a pipe connecting the plasma liquid generating device 1 and the water tank 23 may pass through the wall 21. Thus, the plasma liquid generating device 1 can be installed at indoor (e.g. in the bathroom). The water tank 23 can provide water to the plasma liquid generating device 1. A water output structure 22 is connected to the mixing structure 14, so that the plasma liquid generated by the plasma liquid generating device 1 can be ejected through water output structure 22 for showering or washing. Herein, the water output structure 22 is for example a microbubble shower. In practice, the microbubble shower can achieve the deep cleaning of skin and thus enhance the cosmetic and sterilization effects of the plasma liquid.

As shown in FIG. 6, the plasma liquid generating device 1 can be installed on the outlet of the water tank 23, and the output pipe of the plasma liquid generating device 1 is disposed at any desired place. For example, if the house has three bathrooms, the output pipes can be installed to supply the plasma liquid containing the first type plasma particles (O₃) and the second type plasma particles (NO₃⁻) to the three bathrooms.

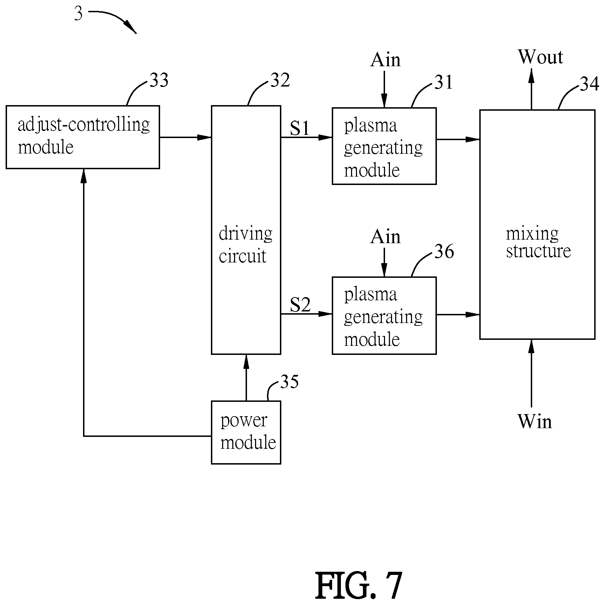

FIG. 7 is a block diagram of another plasma liquid generating device 3 according to an embodiment.

As shown in FIG. 7, the plasma liquid generating device 3 includes a plasma generating module 31, a driving circuit 32, an adjust-controlling module 33, a mixing structure 34, and a power module 35. The technical features of the plasma generating module 31, the driving circuit 32, the adjust-controlling module 33, the mixing structure 34, and the power module 35 can be referred to the plasma generating module 11, the driving circuit 12, the adjust-controlling module 13, the mixing structure 14, and the power module 15 of the previous embodiment, so the detailed descriptions thereof will be omitted.

Different from the plasma liquid generating device 1, the plasma liquid generating device 3 further includes another plasma generating module 36, and the driving circuit 32 can individually drive the plasma generating modules 31 and 36 by the same or different driving methods. In this embodiment, the adjust-controlling module 33 can control the driving circuit 32 to output driving signals S1 and S2 to the plasma generating modules 31 and 36, respectively, thereby controlling the proportions of the first type plasma particles and the second type plasma particles generated by the plasma generating modules 31 and 36.

For example, the plasma generating module 31 includes at least one plasma generator (a first atmospheric-pressure plasma generator), and the plasma generating module 36 includes at least one plasma generator (a second atmospheric-pressure plasma generator). The plasma generating modules (or plasma generators) can be configured with different settings and divided into different groups. For example, the plasma generating module 31 belongs to a first group, and the plasma generating module 36 belongs to a second group. The plasma generator of the plasma generating module 31 of the first group is configured to generate more of the first type plasma particles (O₃),and the plasma generator of the plasma generating module 36 of the second group is configured to generate more of the second type plasma particles (NO₃⁻). Accordingly, the plasma particles generated by the plasma generating module 31 contains more nitrate ions than ozone, and the plasma particles generated by the plasma generating module 36 contains more ozone than nitrate ions. This configuration can properly control the total plasma generated by the plasma generating modules 31 and 36 and adjust the proportion of the plasma particles.

As mentioned above, the plasma generator can be based on the DBD technology. Herein, the plasma generating module 31 of the first group is driven by a high frequency voltage, and the plasma generating module 36 of the first group is driven by a low frequency voltage.

In addition, it is also possible to configure a proper catalyst to control the generation proportion of different plasma particles. In this embodiment, the plasma generating modules 31 and 36 can be configured with different catalysts, which are benefit to generate different plasma particles. For example, the plasma generator of the plasma generating module 31 of the first group is configured with a catalyst benefit to generate the first type plasma particles (O₃), and the plasma generator of the plasma generating module 36 of the second group is configured with another catalyst benefit to generate the second type plasma particles (NO₃⁻).

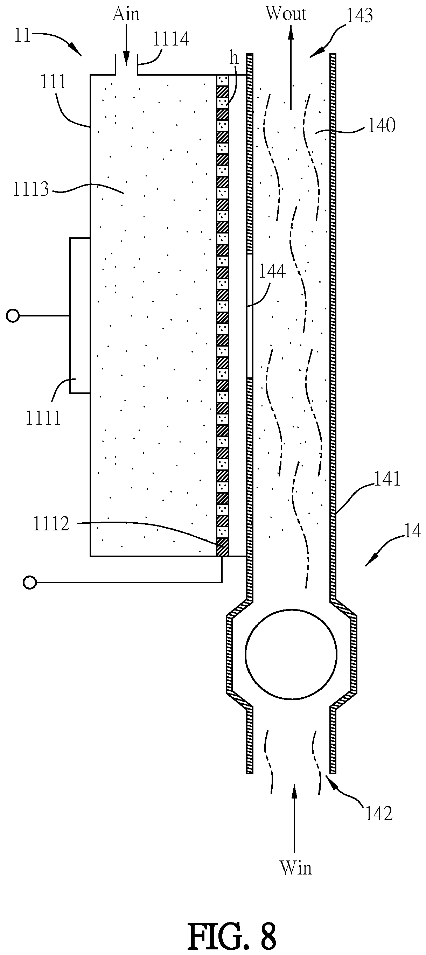

FIG. 8 is a schematic diagram showing a plasma generating module and a mixing structure. The plasma generating module 11 and the mixing structure 14 in FIG. 8 is similar to the relevant elements in FIG. 3. In Fig. 8, the number of the hole 144 of the tube of the mixing structure 14 is one for example. The hole 144 acts as a plasma inlet which allows the plasma particles entering the flow channel 140 from the chamber 1113 of the plasma generator 111. The flow channel 140 is located inside the tube of the mixing structure 14, for example, the tube of the Venturi tube 141. The flow channel 140 is located between the inlet 142 and the outlet 143.

For example, the plasma generator 111 may be an atmospheric-pressure plasma generator, and it comprises an air inlet 1114, a chamber 1113 and at least a hole h as a plasma outlet. Atmospheric-pressure air can enter the chamber 1113 through the air inlet 1114. The plasma particles generated in the chamber 1113 are outputted from the plasma outlet (hole h). The plasma inlet 144 communicates with the plasma outlet (hole h) and the flow channel 140. When the liquid flows through the flow channel 140, the plasma particles are sucked from the chamber 1113 through the plasma outlet (hole h) and the plasma inlet 144 to the flow channel 140, then the plasma particles are mixed in the liquid so as to produce a plasma liquid, and atmospheric-pressure air is sucked through the air inlet 1114 into the chamber 1113. When no air contained within the chamber 1113 is sucked into the flow channel 140, the external air also is not sucked through the air inlet 1114 into the chamber 1113. Namely, when no liquid flows through the flow channel 140, the air (or the air containing the plasma particles) within the chamber 1113 is not sucked into the flow channel 140, and the external air (atmospheric-pressure air) is not sucked through the air inlet 1114 into the chamber 1113.

The power source of the plasma generator 111 may be the hydroelectric generator 151. The hydroelectric generator 151 is configured to output a supply voltage based on the liquid flowing therethrough. The driving circuit 12 is coupled with the atmospheric-pressure plasma generator 111 and the hydroelectric generator 151, configured to transform the supply voltage into a high voltage, and configured to drive the atmospheric-pressure plasma generator 111 to generate the plasma particles. The piping or tubes of hydroelectric generator 151 and the mixing structure 14 communicate with each other. The liquid may flow through the hydroelectric generator 151 first and then flow through the mixing structure 14.

When the liquid flows, the liquid not only drives the hydroelectric generator 151 to power the plasma generator 111 to generate the plasma particles, and also drives the mixing structure 14 to suck the generated plasma particles from the chamber 1113 into the flow channel 140 where the sucked plasma particles are mixed in the liquid. The external air (atmospheric-pressure air) is also sucked through the air inlet 1114 into the chamber 1113, and then the sucked air in the chamber 1113 is ionized to generate plasma particles by the plasma generator 111. When keeping the liquid flowing, the electric power is repeatedly generated by the hydroelectric generator 151, the plasma particles are repeatedly generated in the chamber 1113 and sucked into the flow channel 140 by the mixing structure 14, the sucked plasma particles are repeatedly mixed in the liquid, and the external air (atmospheric-pressure air) is repeatedly sucked through the air inlet 1114 into the chamber 1113.

When no liquid flows, the hydroelectric generator 151 does not operate and does not power the plasma generator 111, the plasma generator 111 is not powered and does not generate plasma particles, the mixing structure 14 does not suck air in the chamber 1113 into the flow channel 140, and the external air (atmospheric-pressure air) is not sucked through the air inlet 1114 into the chamber 1113. Therefore, when no liquid flows, no electric power is generated, no plasma particle is generated, and no air is sucked. In addition to saving electricity is also relatively safe.

In ordinary situation, the pressure within the chamber 1113 and the air outside the air inlet 1114 are equal to atmospheric-pressure. The generated plasma particles do not flow through the air inlet 1114 to the external. Atmospheric-pressure can be the pressure of the unpressured air in daily live and is about 1 atm for example.

For example, the plasma generator 111 may have only one chamber 1113 and only one air inlet 1114. One chamber 1113 only connects one air inlet 1114.

Regarding the plasma generator 111 as discussed above, the amount of air entering the chamber 1113 of the plasma generator 111 depends on the amount of air containing the plasma particles being sucked from the chamber 1113 into the mixing structure 14 (Venturi tube 141). When the flow speed and amount of water increase, the amount of air entering into the chamber 1113 of the plasma generator 111, the amount of plasma generated by the plasma generator 111, and the amount of air containing the plasma particles being sucked into the mixing structure 14 are also increased. In other words, when the amount of water increases, the amounts of plasma particles and plasma liquid are also increased.

Moreover, the plasma generating module 11 and the mixing structure 14 discussed above can be applied to the plasma generating module and the mixing structure in other embodiments.

In the above embodiments, the plasma liquid generating device 1, 3 may be an atmospheric-pressure plasma liquid generating device which comprises at least an atmospheric-pressure plasma generator, a driving circuit and a mixing structure in the above embodiments. The atmospheric-pressure plasma generator comprises an air inlet, a chamber and at least a plasma outlet. Atmospheric-pressure air can enter the chamber through the air inlet. The driving circuit is coupled with the atmospheric-pressure plasma generator to drive the atmospheric-pressure plasma generator to generate plasma particles in the chamber. The mixing structure comprises a flow channel and a plasma inlet. The plasma inlet communicates with the plasma outlet and the flow channel. When a liquid flows through the flow channel, the plasma particles are sucked from the chamber through the plasma outlet and the plasma inlet to the flow channel, then the plasma particles are mixed in the liquid so as to produce a plasma liquid, and atmospheric-pressure air is sucked through the air inlet into the chamber.

In the above embodiments, the adjust-controlling module is coupled with the driving circuit and configured to control the driving circuit to adjust a generation proportion of the plasma particles generated in the chamber by the atmospheric-pressure plasma generator. For example, a generation proportion of the ozone plasma particles and the nitrate ion plasma particles. For example, the plasma particles comprises ozone plasma particles and the nitrate ion plasma particles. When the driving voltage has a high frequency, the generated nitrate ions are more than the generated ozone. When the driving voltage has a low frequency, the generated ozone is more than the generated nitrate ions. The adjust-controlling module can be configured to adjust the frequency of the driving voltage generated by the driving circuit.

As mentioned above, in the plasma liquid generating device, the driving circuit is coupled with the plasma generating module and drives the plasma generating module to generate first type plasma particles and second type plasma particles. Besides, the adjust-controlling module is coupled with the driving circuit and controls the driving circuit to adjust a generation proportion of the first type plasma particles and the second type plasma particles generated by the plasma generating module. The mixing structure mixes the first type plasma particles, the second type plasma particles and a liquid so as to produce a plasma liquid. Accordingly, the plasma liquid generating device can generate a plasma liquid, so that the users may produce plasma liquid in their daily lives.

Although the invention has been described with reference to specific embodiments, this description is not meant to be construed in a limiting sense. Various modifications of the disclosed embodiments, as well as alternative embodiments, will be apparent to persons skilled in the art. It is, therefore, contemplated that the appended claims will cover all modifications that fall within the true scope of the invention.

## Claims

1. A plasma liquid generating device, comprising:
a plasma generating module;
a driving circuit coupled with the plasma generating module and configured to drive the plasma generating module to generate first type plasma particles and second type plasma particles;
an adjust-controlling module coupled with the driving circuit and configured to control the driving circuit to adjust a generation proportion of the first type plasma particles and the second type plasma particles generated by the plasma generating module; and
a mixing structure connecting with the plasma generating module and mixing the first type plasma particles, the second type plasma particles and a liquid so as to produce a plasma liquid.

2. The plasma liquid generating device of claim 1, wherein the first type plasma particles are ozone, and the second type plasma particles are nitrate ions.

3. The plasma liquid generating device of claim 2, wherein the driving circuit is configured to output a driving voltage to the plasma generating module;
wherein when the driving voltage has a high frequency, the generated nitrate ions are more than the generated ozone in the generation proportion; and
wherein when the driving voltage has a low frequency, the generated ozone is more than the generated nitrate ions in the generation proportion.

4. The plasma liquid generating device of claim 3, further comprising:
a power module configured to output a supply voltage to the driving circuit, wherein the driving circuit comprises:
a voltage frequency adjustor configured to generate a modulated frequency according to an adjusting signal outputted from the adjust-controlling module;
a voltage convertor coupled with the power module and the voltage frequency adjustor and configured to output the driving voltage according to the supply voltage and the modulation frequency; and
a booster configured to boost the driving voltage and output the driving voltage to the plasma generating module.

5. The plasma liquid generating device of claim 4, wherein the power module comprises a hydroelectric generator, and the liquid flows through the hydroelectric generator so as to enable the hydroelectric generator to output the supply voltage.

6. The plasma liquid generating device of one of claims 1-5, wherein the plasma generating module comprises at least an atmospheric-pressure plasma generator, the atmospheric-pressure plasma generator comprises a first electrode and a second electrode, the first electrode is disposed opposite to the second electrode, the first electrode has a plate shape, the second electrode has at least a hole, and the first type plasma particles and the second type plasma particles flow into the mixing structure through the hole.

7. The plasma liquid generating device of claim 6, wherein the mixing structure comprises a Venturi tube and an inlet and an outlet connecting to the Venturi tube, and the second electrode is disposed on the Venturi tube close to the outlet.

8. The plasma liquid generating device of one of claims 1-7, wherein the plasma generating module comprises:
a first atmospheric-pressure plasma generator configured to generate a group of plasma particles mainly containing the first type plasma particles; and
a second atmospheric-pressure plasma generator configured to generate a group of plasma particles mainly containing the second type plasma particles.

9. The plasma liquid generating device of one of claims 1-8, further comprising:
a waterproof housing for accommodating the plasma generating module, the driving circuit and the mixing structure, wherein the adjust-controlling module is disposed on the waterproof housing.

10. A compact automatic plasma liquid generating device, comprising:
at least an atmospheric-pressure plasma generator;
a hydroelectric generator configured to output a supply voltage based on a liquid flowing therethrough;
a driving circuit coupled with the atmospheric-pressure plasma generator, configured to transform the supply voltage into a high voltage, and configured to drive the atmospheric-pressure plasma generator to generate ozone plasma particles and nitrate ion plasma particles;
an adjust-controlling module coupled with the driving circuit and configured to control the driving circuit to adjust a generation proportion of the ozone plasma particles and the nitrate ion plasma particles generated by the atmospheric-pressure plasma generator; and
a mixing structure connecting with the atmospheric-pressure plasma generator and configured to mix the ozone plasma particles, the nitrate ion plasma particles, and the liquid so as to produce a plasma liquid; and
a waterproof housing for accommodating the atmospheric-pressure plasma generator, the hydroelectric generator, the driving circuit and the mixing structure, wherein the adjust-controlling module is disposed on the waterproof housing.

11. An atmospheric-pressure plasma liquid generating device, comprising:
at least an atmospheric-pressure plasma generator, comprising an air inlet, a chamber and at least a plasma outlet;
a driving circuit coupled with the atmospheric-pressure plasma generator to drive the atmospheric-pressure plasma generator to generate plasma particles in the chamber; and
a mixing structure comprising a flow channel and a plasma inlet, wherein the plasma inlet communicates with the plasma outlet and the flow channel, when a liquid flows through the flow channel, the plasma particles are sucked from the chamber through the plasma outlet and the plasma inlet to the flow channel and mixed in the liquid so as to produce a plasma liquid, and atmospheric-pressure air is sucked through the air inlet into the chamber.

12. The atmospheric-pressure plasma liquid generating device of claim 11, wherein the atmospheric-pressure plasma generator comprises a first electrode and a second electrode, the first electrode is disposed opposite to the second electrode, the first electrode has a plate shape, and the second electrode has at least a hole as the plasma outlet.

13. The atmospheric-pressure plasma liquid generating device of one of claims 11-12, wherein the mixing structure comprises a Venturi tube and an inlet and an outlet connecting to the Venturi tube, and the second electrode is disposed on the Venturi tube close to the outlet.

14. The atmospheric-pressure plasma liquid generating device of one of claims 11-13, wherein the driving circuit is configured to output a driving voltage to the atmospheric-pressure plasma generator, and the plasma particles comprises ozone plasma particles and nitrate ion plasma particles;
wherein when the driving voltage has a high frequency, the generated nitrate ions are more than the generated ozone;
wherein when the driving voltage has a low frequency, the generated ozone is more than the generated nitrate ions;
wherein the atmospheric-pressure plasma liquid generating device further comprises an adjust-controlling module coupled with the driving circuit and configured to control the driving circuit to adjust a generation proportion of the ozone plasma particles and the nitrate ion plasma particles generated in the chamber by the atmospheric-pressure plasma generator.

15. The atmospheric-pressure plasma liquid generating device of one of claims 11-14, further comprising:
a hydroelectric generator configured to output a supply voltage based on the liquid flowing therethrough;
wherein the driving circuit is coupled with the atmospheric-pressure plasma generator and the hydroelectric generator, configured to transform the supply voltage into a high voltage, and configured to drive the atmospheric-pressure plasma generator to generate the plasma particles.
